# EUROPEAN PATENT SPECIFICATION

(11) **EP 0 380 586 B1**
(45) Date of publication and mention of the grant of the patent: **08.12.1999**
(21) Application number: 88909936.2
(22) Date of filing: 21.09.1988
(51) Int. Cl.: A61B 1/06

(54) **CATHETER SYSTEM FOR IMAGING**
KATHETERSYSTEM ZUR ABBILDUNG
SYSTEME DE CATHETER POUR FORMATION D'IMAGES

(30) Priority: 24.09.1987 US 100714
(43) Date of publication of application: 08.08.1990
(73) Proprietor: MASSACHUSETTS INSTITUTE OF TECHNOLOGY, Cambridge, MA 02139 (US)
(72) Inventor: KITTRELL, Carter, Cambridge, MA 02139 (US); FELD, Michael, S., Newton, MA 02168 (US)
(74) Representative: Greenwood, John David
(86) International application number: US8803257
(87) International publication number: WO8902718

(56) References cited:
- US-A- 4 163 148
- US-A- 4 494 823

## Description

### Technical Field

This invention relates to a system and method in which optical fibers are provided within a catheter which is coupled to a spectrometer with an imaging detector and diagnostic light is used to create a spectral image of the target tissue.

### Background of the invention

Optical spectroscopy is proving to be a powerful tool for probing the type and condition of tissue. Images of tissue may be recorded remotely, and in vivo, using flexible coherent bundles of optical fibers. Spectra may be recorded in a conventional way using a scanning monochromator. But this is time consuming and inefficient in using the often weak signals associated with tissue spectroscopy, especially where fluorescence excessive intensity may damage tissue thermally and excessive fluence may damage tissue photochemically or by other means. A spectrograph fitted with an imaging device, such as a linear diode array, charge coupled device (CCD), charge injection device (CID), vidicon or other such imaging device, converts the entire dispersed spectrum into an electronic signal so that all the desired light is detected simultaneously. As the spectrograph may be connected to an optical fiber, the emitted spectrum is recorded. The other end of the fiber may be placed in tissue or at the bottom of a well or in other remote locations, to collect the light carrying information about its environment. However, this device records only a spectrum, not an image. Similarly, coherent bundles of optical fibers transmit images from remote locations, which are recorded on similar electronic imaging devices for video display and storage. However, combining spectral analysis and imaging has proved more difficult.

There is a need for a means to spectrally record the entire image simultaneously. Instead of using a single optical fiber consider the use of an image from a coherent bundle of optical fibers that is placed at the entrance of a spectrometer. If the image was monochromatic, then a monochromatic image would also form on the imaging detector at a certain location, depending on the position of the grating, or other dispersing device. A spectrograph with an adequate flatness of field is assumed here. If a second color was added to the image, then a second image would form on the detector, displaced from the first image, but in some part overlapping the first, depending on the amount of dispersion. Any given detector element might be detecting light from one part of the image of one color, or another part of the image, of a different color. The output would be ambiguous. As more colors are added to the input image, a single element would detect more and more different parts of the image in different colors, with no intrinsic way to sort out the mixed data. The imaging detector will obtain a quite unsuitable spectrally blurred or smeared image.

Fluorescence and laser induced fluorescence has been used to diagnose tissue type and condition. A wavelength of 480 nm excited fluorescence in the range of 500-650 nm has been used, whereby the ratios of peak and valley signals were useful to distinguish atheromateous plaque from normal artery wall. In Alfano, visible laser excitation induced fluorescence in cancerous and normal tissue, with emission over a range of 500-700 nm, which showed differences in the spectra for the tissues. However, other wavelengths of excitation and detection may be useful. Fluorescence emission characteristics of a particular chromophore tend to occur at the same wavelength, for a broad range of excitation wavelengths.

A different type of spectroscopy, termed Raman spectroscopy, depends on a frequency shift from the exciting laser line. The Raman spectral peaks will occur at a fixed frequency separation from the laser line, and, in principle, occur for any excitation wavelength. It is the frequency difference that is the most distinguishing feature. Raman spectroscopy has been used for identifying a wide range of substances such as molecules in solution or in flames.

US-A-4,648,892 discloses a system for, and a method of, examining tissue according to the preambles of claims 1 and 13, resepectively. The system and method of the present invention is characteried by the features of the characterizing portions of these claims.

In accordance with the invention, the optical fibers, the spectrometer, and the detector will be configured in a novel way so that information adequate to construct both image and spectra will be obtained.

Consider a specific embodiment and number of optical fibers described below; but the invention need not be limited in this manner. A coherent optical fiber bundle comprised of 10,000 fibers in a square array, having 100 columns of fibers side by side, with each column containing 100 individual fibers in vertical array. Each fiber has a 10 micrometer core and a 20um outer diameter, including cladding. The coherent bundle forms a square with a 2mm cross section at the input or distal end. At the output end, the columns are separated one from another and spaced to a distance of 500µm to form a rectangular array 2mm by 50mm. This array, or an image of the array is placed at the entrance of low-dispersion spectrometer which is corrected to have adequate flatness of field and minimize other abberations. For no magnification, the dispersion is chosen so that the spectral region of interest does not exceed 500µm. This spectral region may be limited with filters to avoid overlap. Dispersion in the horizontal plane will be assumed throughout this description, but it need not be limited to this plane. Impinging on a 2-dimensional image detector of adequate dimension (at least 50mm wide in this case), is such that the dispersed spectrum of each column will not overlay that of the next column. For a detector with a 20µm square element, each pixel element, corresponding to each optical fiber, will be dispersed over 25 detector elements, subdividing the spectrum into 25 resolution elements. Thus, each of the 10,000 optical fibers will couple to a separate 25 elements of the detector which has 250,000 elements, so that each spectrum may be recorded individually without overlapping another spectrum.

For a more compact system with a square detector array, columns of fibers could be placed one below another in groups. For example, if 5 columns from the coherent bundle were combined into a single column 500 fibers each 10mm long, and there were 20 such columns spaced 500µm apart, this would form a square array 10x10mm and could be projected onto a square detector with or without magnification. Without magnification each image pixel would be dispersed over 25 detector elements. A larger number of detector elements, and appropriate adjustments of column spacing and magnification would allow for more spectral resolution elements per pixel.

Another arrangement of optical fibers would consist of subdividing the square array into a square block rather than columns. For example, a 5x5 block of fibers could be relocated, and arranged into a 25 fiber column. The image would be dispersed onto a square portion of the detector 25 elements high by 25 elements wide. The adjacent block of optical fibers would be imaged onto a corresponding adjacent block of the detector. This would minimize crosstalk problems between widely separated parts of the image, as adjacent parts of the image are observed by adjacent parts of the detector. With this type of arrangement, a lower resolution image could be obtained if desired. The block of optical fibers and corresponding block on the detector might be treated as a single pixel. Of course, any arrangement of optical fibers of the distal end might bp matched in many ways to the image detector.

Special optical coupling elements may be installed between the output of the optical fiber bundle, the spectrometer and the detector. Anamorphic elements, such as cylindrical lenses or prisms, can expand the image more in one dimension than another. The rectangular 2mm x 50mm image previously described could be changed to a rectangle of different dimensions, or even a square. Likewise, the round image of a single optical fiber can be made oblong to match a rectangular element on a detector array.

An image may also be spectrally resolved with a somewhat different type of apparatus. The image from the optical fibers is collimated and sent through a travelling Michaelson interferometer. A single pixel undergoes constructive and destructive interference as the travelling mirror moves; the recorded spectrum as a function of time is converted by Fourier transform to a wavelength spectrum. If however, the detector is replaced by an imaging device, such as a CCD, then every pixel is subject to the interferometer. The imaging device needs to be "read out" many times as the mirror scans, so as to provide enough data for an accurate transform to be obtained. As an example, the CCD would be read out entirely twenty times for each wavelength of travel of the mirror. The time to acquire a spectral image may become significant. Also, substantial computing power should be needed to do the Fourier transform of the data for thousands of pixels in a small amount of time.

Another system which can spectrally filter an image from an optical fiber bundle would be comprised of filters and beamsplitters. The light from the image may pass through a series of beam splitters to fall on one or more imaging detectors. Dichroic beamsplitters will separate the light into various colors with minimal losses. Absorbing filters may also be interposed in the light path to select wavelengths, however these are less efficient. Multiple images, each of a different color, may impinge on a single imaging detector.

It is anticipated that there will be a broad range of uses for this type of spectroscopic imaging device, both within and outside of the medical field.

We have found ultraviolet excited laser induced fluorescence provides a useful diagnostic for arterial tissue distinguishing normal from plaque. Experiments used wavelengths from 220 nm to 330 nm for excitation, with detection ranging from close to the laser wavelength to 750 nm. Several useful laser induced fluorescence diagnostics were found. Two peaks and a valley in the range of 370-470nm are useful for distinguishing normal and plaqued artery. Even after laser ablation damage, the valley of about 420 nm persists. It is likely due to the Soret band of porphyrin, probably from heme. These features should also be useful for identifying other types of tissue as well. Weak LIF emission also occurred in the range 600-700 nm with a peak at 650 nm, for the wide range of ultraviolet excitation. Also, ultraviolet wavelengths shorter than 320 nm excite a broad fluorescence probably due to tryptophan, which for 220-280 nm excitation ranges from a spectrum in the range 290-380 nm with a maximum of about 335 nm. The intensity, and the fluorescence lifetime (typically about 3 nsec) varies with tissue type and may provide a useful diagnostic.

A Raman peak useful for diagnostic occurs at about 3300cm⁻¹ ± 150cm⁻¹. Although excitation wavelengths in the range 220-228 nm were used, Raman scattering occurs for all excitation wavelengths; it is the frequency shift that is the distinguishing characteristic. Plaque showed a much stronger Raman signal than did the healthy arterial media. The frequency shift remained relatively constant as the excitation frequency was changed, proving that the spectrum is a Raman process, and not LIF. This signal persisted after laser albation damage, and was useful in determining that when plaque layer was penetrated, the underlying healthy media gave a weaker signal. This change in Raman signal strength could be used as a feedback control diagnostic to signal that plaque has been removed, and that tissue ablation may be halted.

### Brief Description of the Drawings

Figure 1 is a broken longitudinal cross-section of the imaging catheter, spectrometer and a block diagram of the computer and outputs, illustrating a preferred embodiment of the invention.
Figure 2 is a cross-sectional view of the imaging catheter.
Figure 3 is an optical schematic illustrating target illumination by the same optical fibers used to collect the emitted or reflected light, and illustrates optical imaging of the proximal end of the catheter onto the spectrometer aperture.
Figure 4 is an optical schematic showing the use of dual detection systems.
Figure 5 is an optical schematic showing the use of a Michaelson interferometer.
Figure 6 is an optical schematic illustrating the use of a lens to image the target onto the distal end of the catheter.

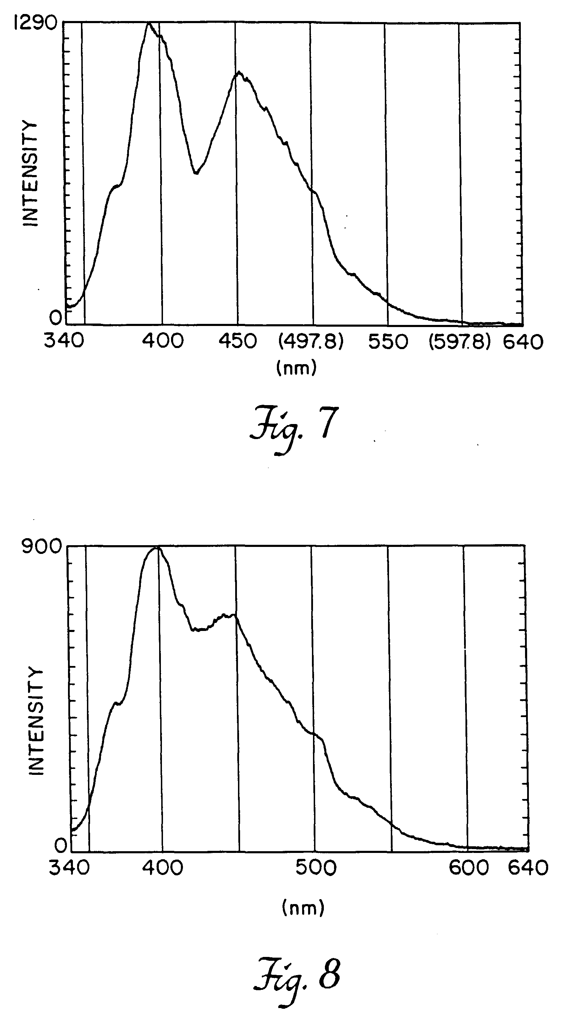

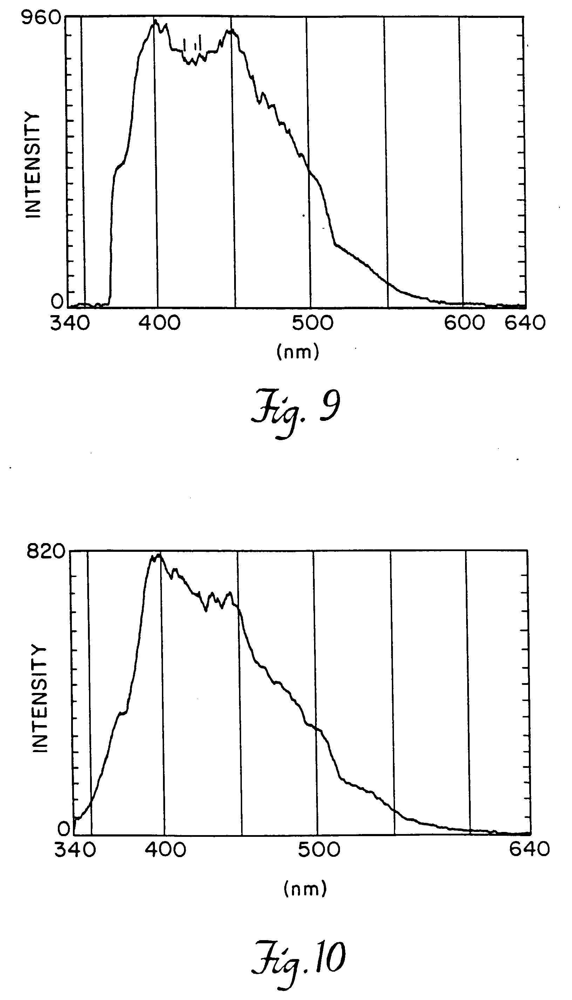

Figure 7 shows fluorescence of a normal aorta artery wall stimulated by 329nm excitation. The left hand scale (ordinate) is intensity of fluorescence signal (figures 7-10 use the same conditions).
Figure 8 shows plaque fluorescence under the same conditions of Fig. 7. This is a soft plaque, mainly fiberous and fatty. The peaks have shifted slightly in wavelength and in the height ratio; but the valley is much less deep.
Figure 9 shows the spectrum whereby an argon ion laser, using multiple shots, has ablated through the 1-2mm plaque layer to reach the normal media beneath. Total dosage was about 5-10J/mm². The spectrum of the tissue at the bottom of the crater is shown. The spectral shape is somewhat altered, but a valley at about 425nm persists.
Figure 10 shows an adjacent region ablated by the argon ion laser, fewer exposures were made so the plague was not penetrated. This spectrum is clearly distinguishable from Figure 9, showing that this is a useful diagnostic for determining when the plague layer has been penetrated.

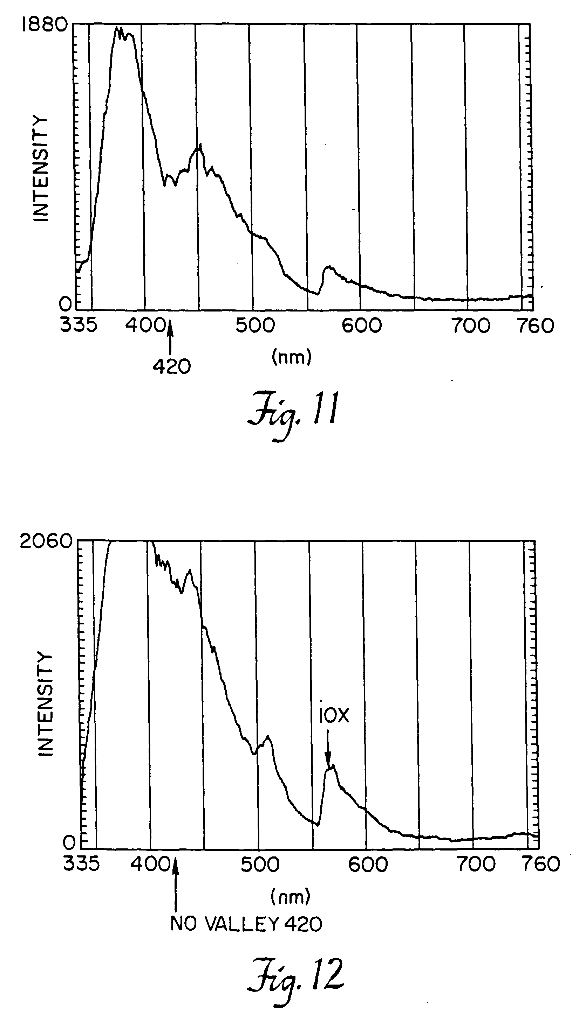

Figure 11 shows fluorescence from normal aorta using 320nm excitation.
Figure 12 shows fluorescence from plaque under the same conditions. This shows that useful diagnostic information can be obtained for various excitation wavelengths.

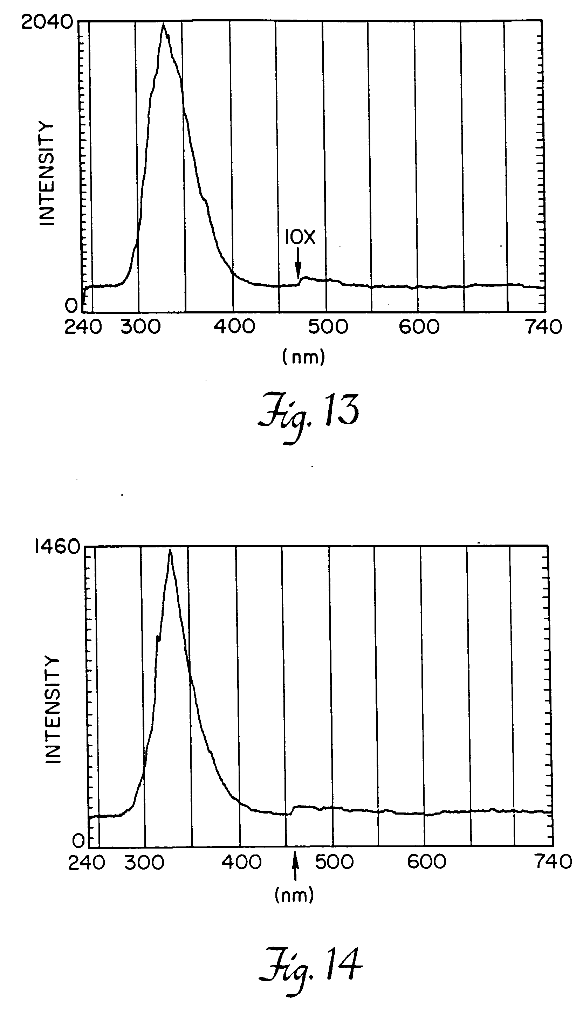

Figure 13 shows normal artery excited by 220nm, yielding a fluorescence peak of about 330nm, most likely due to tryptophan. A fluorescence lifetime of about 3nsec was observed; this may also yield useful diagnostic information.

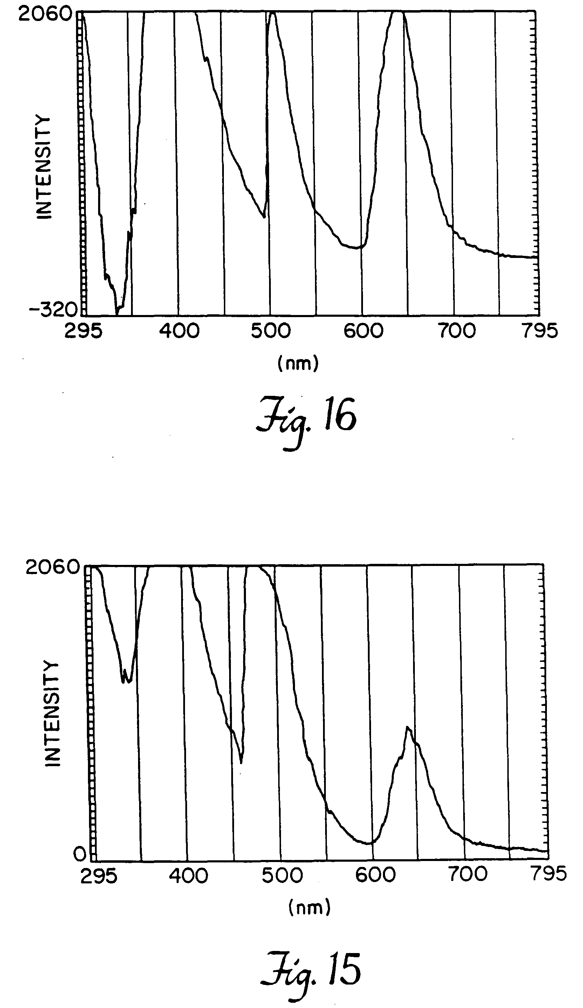

Figure 14 shows plague on the same artery sample, under the same conditions. As for Figure 13, the peak has less intensity. Any UV wavelength shorter than 330nm excites this fluorescence.
Figure 15 using 280nm excitation, a weak peak at 650nm is seen. The detection sensitivity is high so the tryptophan peak is off scale. This is for plaque.
Figure 16 shows a fluorescence spectrum for normal tissue, under the same conditions as for Figure 15 showing greater intensity for the 650nm peak.
Figure 17 shows a Raman peak at about 244nm. The laser line is about 225nm. (A correction of -2nm is needed for all following Figures 17-22). There is an intensity scale change at about 233nm. This spectrum is for soft plaque, fiberous and fatty. The frequency shift is about 3,320cm⁻¹. This corresponds to an O-H stretching frequency, or possibly that of N-H.
Figure 18 shows a spectrum for normal arterial media on the same tissue sample. The Raman peak is much weaker.
Figure 19 shows a spectrum of calcified plaque which has been irradiatedwith about 2 Joules/mm² from an argon ion laser, 514nm. The strong Raman peak at 244nm is present, with a frequency shift of 3,320cm⁻¹.
Figure 20 shows a different excitation wavelength for one laser 222nm; the Raman peak has moved also proving that this is Raman and not fluorescence emission. The difference in frequency is about 3,290cm⁻¹, which is unchanged within experimental error..
Figure 21 shows a Raman spectrum of plaqued aorta artery wall whereby the plaque has been ablated away as described for Figure 9. The Raman peak at about 242nm is weak, indicating that the overlying plaque has been removed.
Figure 22 shows Raman spectrum of ablated plaque which was not penetrated, similar to that described for Figure 10. The Raman peak at about 242nm is strong, indicating that there is still plaque at the bottom of the crater. A weak "shoulder" peak is seen at about 232nm corresponding to about a 1600cm⁻¹ Raman shift which could be a carbonyl stretching frequency; this may also have useful diagnostic properties, but better laser light rejection will be needed to examine it.

### Detailed Description of the Invention

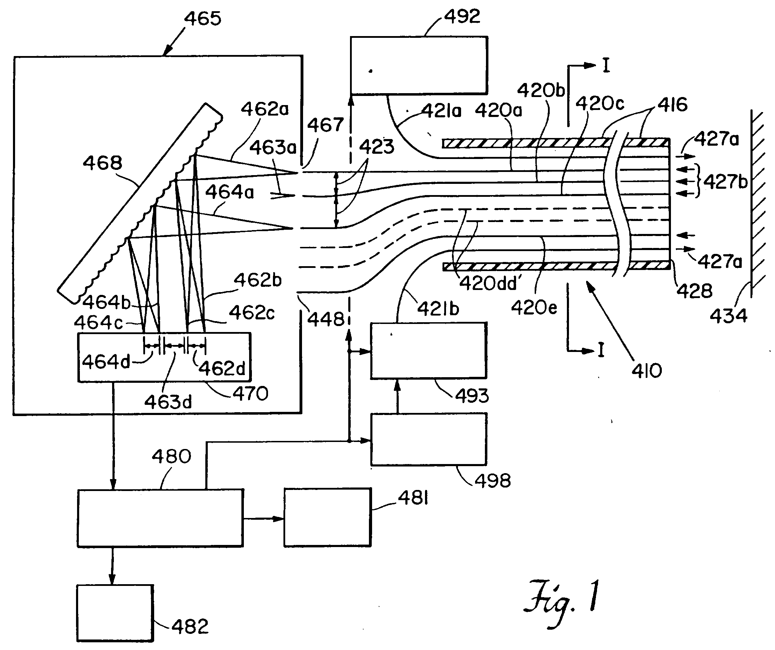

Figure 1 shows a preferred embodiment of the invention. The catheter 410 is shown in broken longitudinal section. It has a distal end 428 and a proximal end 448 and contains 10,000 imaging optical fibers in a 100 by 100 square array. Optical fibers 420 a,b,c,e are shown, with 420 dd' indicating the large number of additional optical fibers in the array. Illuminating optical fibers 421 a,b are coupled to a laser source 492 or a conventional light source 498 with wavelength selection 493 comprised of filters, a monochromator, or similar device. Illuminating light 427 a emerges from illuminating fibers 421 a,b and falls on the target tissue such as sample 434, which may or may not be in contact with the catheter. Light 427 b returning from the sample is collected by the fibers 420 a-e to be delivered to the spectrometer 465.

Since this is a cross-sectional view, each optical fiber illustrated actually represents a column of 100 optical fibers. These columns of fibers are physically separated by a distance indicated by arrow 423 at the proximal end 448 of the catheter and all are placed at the entrance aperture 467 of the spectrometer 465. The emitted rays of light from each column of fibers 420 a-e fall on the grating 468 and are dispersed. The grating may be self focussing as shown, or the spectrometer 465 may contain additional optics to produce the image at the two dimensional detector array 470. Because of the physical separation 423 between fibers, the spectrally dispersed images 462d, 463d, and 464d of the fibers will be physically separated on the detector array 470. Light rays 462a spreading from fiber 420a are dispersed in the horizontal plane with the longer wavelength rays 462c and shorter wavelength rays 462b impinging on the detector 470 over a range of 462d. Similarly, rays 464a from fiber 420c are dispersed to long 463d. These spectral ranges do not overlap, so that spectra are recorded separately.

From detector 470, the electronic signals go to the computer and controller 480, where they are processed and displayed as a spectral map on terminal 482 or output as hard copy on 481. The computer may also control light source 498, scannable monochromator or changeable filter wavelength selector 493, or laser light source 492. Both excitation and emission spectra may be obtained. The computer 480 records the spectrum from each optical fiber to construct the spectral image; the light from each fiber 420 a-e represents a pixel of data for the image. With suitable algorithms, the spectra are compared to stored spectra and converted to diagnostic information about each pixel, such as tissue type, and presence or absence of disease. An image showing such a condition is displayed on the monitor 482, or printed out on 481.

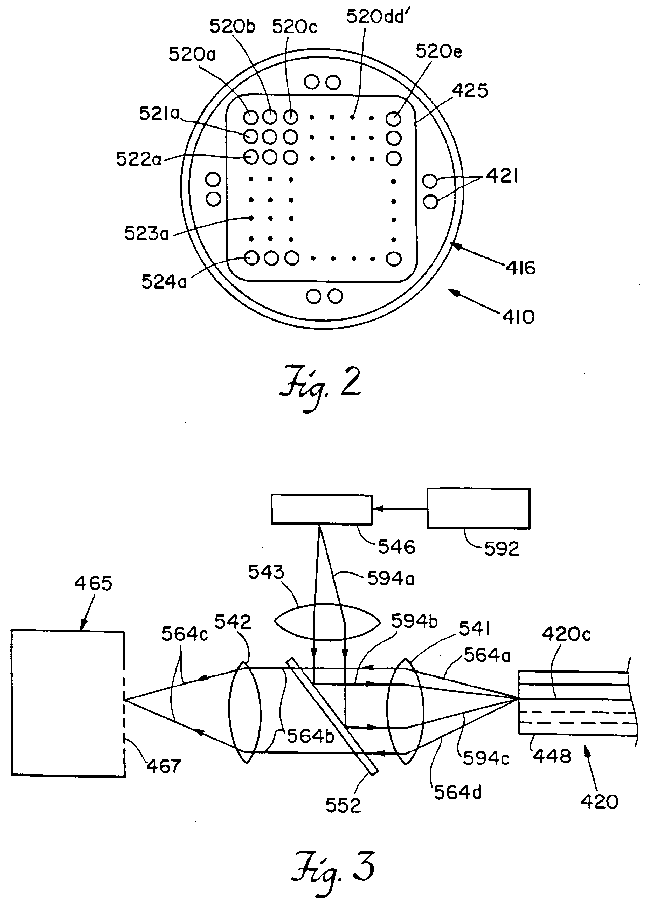

Figure 2 shows a cross section of the optical fiber catheter 410 in a preferred embodiment, with the array at 100 columns of fibers containing one hundred fibers each. The first column comprises the fibers 520a, 521a, 522a, 524a, and with more fibers indicated by dotted line 523a. The second column has fiber 520b at the top, and the third column has fiber 520c, the last column has fiber 520e at the top. Additional columns are indicated by dotted lines 520 d-d'. Illumination fibers 421 are shown at the periphery, although they may also be located between the columns. An optional inner sleeve surrounds the fibers. The whole is contained in an outer tube or catheter 416.

Figure 3 is an alternate embodiment where by the proximal end 448 of the catheter is imaged onto the aperture 467 of the spectrometer 465. Lenses 541 and 542 make the image, but mirrors or other optics may also be used for this purpose. Rays 564a from the optical fibers are collimated 564b and focussed 564c. Optional beamsplitter 552, which may be dichroic or partially reflecting, allows illuminating light 594a from source 592 and coupling selector 546 to be coupled directly into the optical fiber bundle 520. The rays 594b are focussed 594c onto the proximal end of the catheter 448. Selector 546 may contain optics and beam directions to allow coupling into any or all of the optical fibers 420 a-e in the catheter 410.

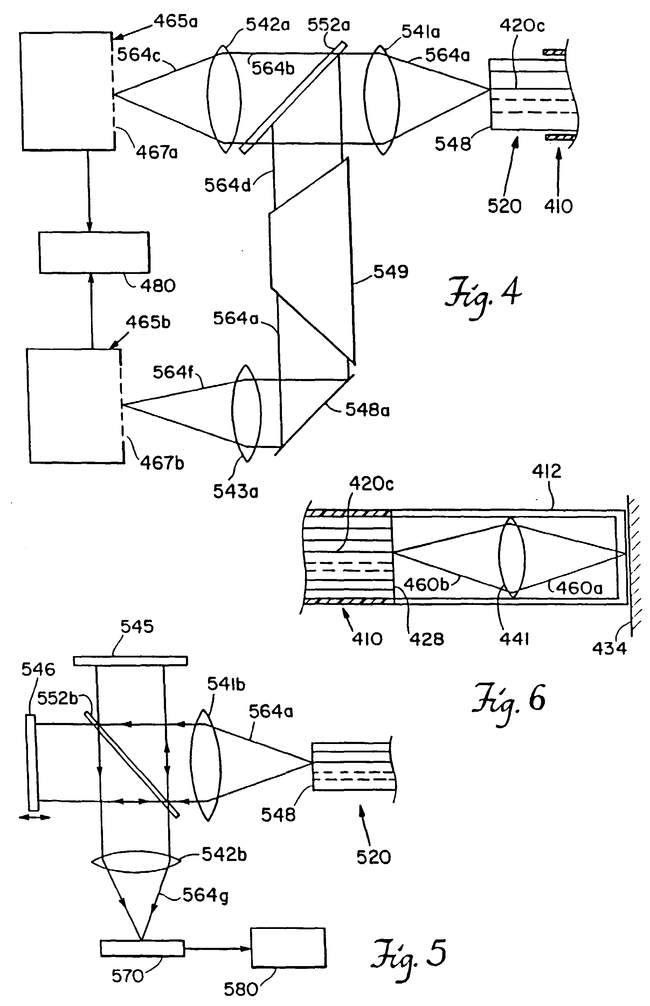

Figure 4 shows an alternate embodiment using two spectrometers. The light emerges from the proximal end 548 of the catheter 410. The columns of fibers in the bundle 520 need not be separated. Rays 564a are collimated 564b by lens 541a and are divided by partially reflecting beamsplitter 552a. Some of the light rays 564c form an image on the aperture 467a of spectrometer 465a. Some of the light rays 564d passes through image rotating prism 549 or similar optical system. The rays 564e now rotated through an angle such as 90°, reflect from optional mirror 548a and are converged 546f by lens 543a to form an image on aperture 467b of spectrometer 465b. As in Figure 1, the two spectrometer detectors are connected to a computer where the data from the two spectrally dispersed images can be processed.

Figure 5 shows an alternate embodiment using a Michaelson interferometer. The proximal end 548 of the optical fiber bundle 520 has light rays 564a which are collimated by lens 541b, entering into Michaelson-type interferometer comprised of beam splitter 552b, fixed mirror 545 and movable mirror 546. The exiting light is focussed by lens 542b, and the rays 564g are imaged onto two dimensional detector 570. The computer 580 is capable of performing Fourier transforms of the data from all of the elements on the detector. The detector 570 need have only as many elements as there are optical fibers in the bundle 520, since this is a non-dispersive system.

Figure 6 shows an alternative embodiment with collection optics at the distal end of the catheter. Lens 441 collects light rays 560a emanating from tissue 434 and focusses the rays 460b onto the distal surface 428 of the catheter 410, for illustration, the rays corresponding to optical fiber 420c are shown. An optional optical shield 412 may be used to protect the optics.

In alternate embodiments, any number of optical fibers may be arrayed in the catheter in a square, hexagonal, circular, or other patterns. In Figure 4, the beam is divided into two components to be directed into two spectrometers. Additional beamsplitters or mirrors may be used to further subdivide the beam before going to the spectrometers. Other coupling optics, represented by those in Figure 3, may be used to magnify the image or expand it anamorphically to better suit the shape of the detector. Columns of optical fibers may be combined or separated so that the number of columns at the proximal surface is not the same as the number in the catheter, and the number of fibers within a column may also be changed. This will better match the geometry of the fiber array to that of the detector. More than one detector array may be used to obtain adequate coverage of the dispersed spectral image. This device may be used for medical imaging on any application where it is desirable to obtain both spectral and spatial imaging.

Whole arterial wall was placed in a capped fused silica cuvette, flush against the face, with moist clean paper behind the tissue to prevent drying. Pulsed ultraviolet laser light was used to excite fluorescence in the tissue. The fluorescence was collected with fused silica optics and passed through a scanning double monochromator onto a photomultiplier. After gated integration, the signal was stored in a computer.

Excitation with about 329nm generated fluorescence from the laser wavelength to the sensitivity limit of the photomultiplier at about 700nm. Peaks were observed at about 395 and 450nm ± 10nm with a valley at 425 nm. The valley was deep for normal artery wall and shallow for plaque. Ablation damage from high power exposure with an argon ion laser upon the tissue altered the spectra, making the valley shallower in both cases. But differences remained, showing that diagnostic difference persists after laser damage. The valley is probably due to reabsorption by the Soret band of porphyrin, probably from heme. Shorter wavelength excitation using wavelengths as short as 220nm excited strong fluorescence at 335nm, most likely from tryptophan. A fluorescence lifetime of a few nanoseconds was observed for this emission and for other emission wavelengths as long as 550nm. The intensity and time resolved fluorescence characteristics of these spectra should provide tissue diagnostic information. Weak emission at 600-700nm, with a peak about 650nm, also appearing to be a useful diagnostic and occurs for all ultraviolet excitation.

For fluorescence spectroscopy, the molecular chromophore is put into an excited electronic state by absorption of a photon of light. The emission is characteristic of the particular chromophore; the peak wavelength tends to remain constant when the excitation wavelength is changed. But excitation wavelength must match the chromophore absorption band.

Raman spectroscopy, which probes the vibrational energy levels of a molecule, eliminates this problem as almost any wavelength can be used. Due to the vibrational transitions of molecules contained in the material under study, light incident upon the material is inelastically scattered thereby transferring energy to the molecule and typically causing a decrease in the frequency of the scattered light.

The use of Raman spectroscopy for diagnostic purposes in distinguishing artery wall from atheromateous plaque via an optical fiber bundle disposed in a catheter is claimed.

Excitation at shorter wavelengths, particularly around 220nm, yielded a Raman signal with about a 3300 ±150 wavemeter Stokes shift. This Raman signal was much stronger for plaque than for healthy artery wall. Since the shift is constant and independent of the excitation wavelength, it is a Raman signal. Such Raman signals should occur for all excitation wavelengths, although it is more difficult to observe when there is strong fluorescence emission also. The Raman signals Persisted after the tissue was ablated with a high power argon ion laser. When the argon laser ablated an overlying layer of plaque, exposing normal media underneath, the Raman signal was substantially reduced, indicating that this signal is a useful diagnostic for determining when the plaque layer has been penetrated.

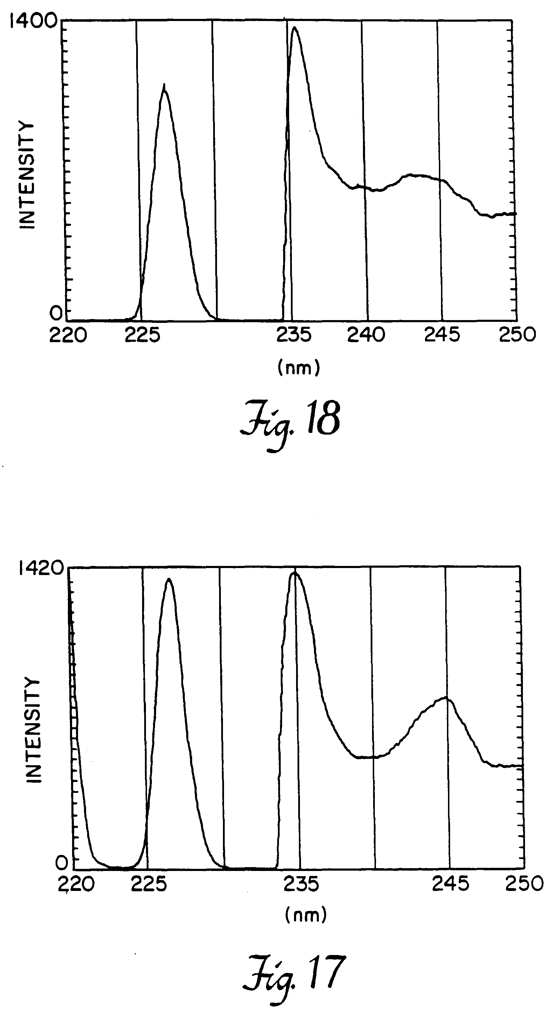

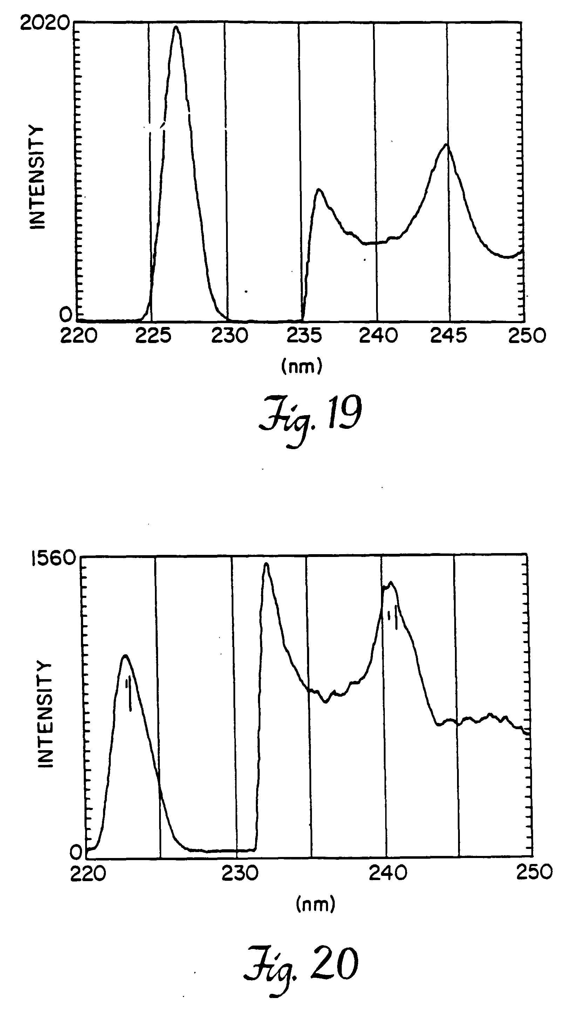

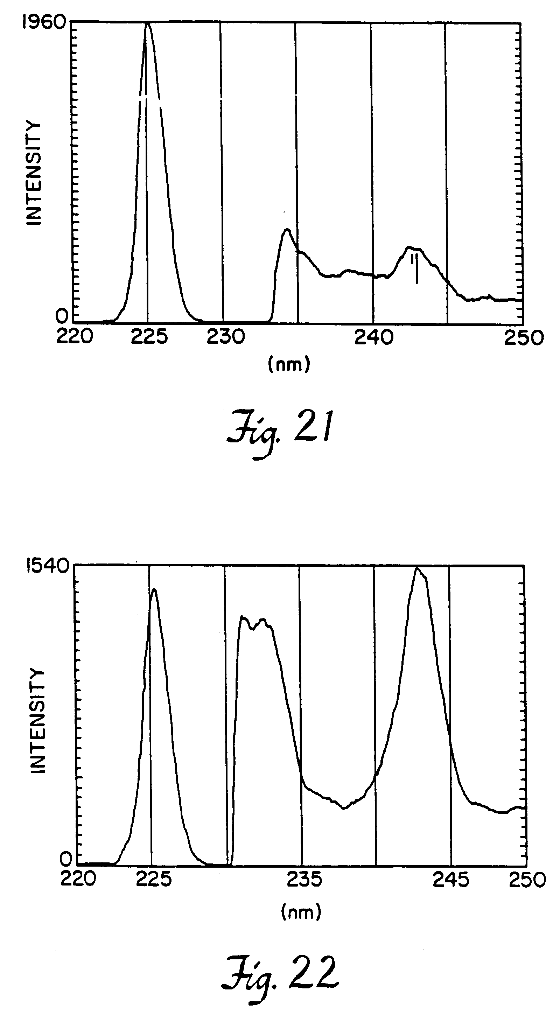

The Figures 17-22 show the data which illustrates the use of the Raman diagnostic for distinguishing plaque from underlying media. For the diagnostic to be useful for monitoring an ablation process, spectral differences should be present after ablation. Although the spectra of normal arterial tissue change to some extent after ablation has occurred, the plaque and healthy media can still be distinguished. In addition, the changes which occur as a result of ablation can be useful as a tissue damage monitor. Most important, however, is a means to determine when the plaque has been penetrated, and that media has been reached so that a signal can be generated to warn the operator that the process should stop; or the signal could be used as a feedback control to terminate laser firing through the particular optical fiber automatically.

Since the Raman scattering generally occurs from a relatively simple functional group, in this case the hydroxyl group (OH) or perhaps a nitrogen-hydrogen (NH) bond, it is probably quite photochemically and thermally stable, and resists laser damage. This is unlike the case for laser induced fluorescence which generally involves molecular chromophores and are more likely to suffer such damage. This makes the Raman spectra a promising new diagnostic tool for monitoring a tissue ablation or treatment process.

Raman scattering occurs for many frequencies as well. A signal is observed of about 1600cm⁻¹, but is partially obscured by scattered laser light passing through the scanning monochromator. This frequency shift may also prove to be a useful diagnostic.

In order to more accurately determine the frequency shift, the monochromator was scanned over the laser line, with the light-detecting photomultiplier adjusted to low gain. About 5-8 nm past the laser line (longer wavelength), the photomultiplier gain is greatly increased, resulting in a sharp rise in the signal on each Raman scan.

The following data taken from a number of scans prove that the peak is a Raman peak, and is not due to laser induced fluorescence.

| Spectrum Number | Laser Wavelength cm⁻¹ | Raman Shift cm⁻¹ |
|---|---|---|
| 1 | 43,971 | 3263 |
| 2 | 43,971 | 3298 |
| 3 | 43,191 | 3266 |
| 4 | 44,105 | 3197 |
| 5 | 44,165 | 3343 |
| 6 | 44,205 | 3228 |
| 7 | 44,245 | 3268 |
| 8 | 44,245 | 3177 |
| 9 | 44,420 | 3325 |
| 10 | 44,440 | 3314 |
| 11 | 44,440 | 3348 |
| 12 | 44,440 | 3365 |
| 13 | 44,504 | 3318 |
| 14 | 44,524 | 3167 |
| 15 | 44,524 | 3320 |
| 16 | 44,543 | 3323 |
| 17 | 44,823 | 3295 |
| 18 | 44,843 | 3263 |
| 19 | 44,843 | 3332 |
| 20 | 44,843 | 3366 |
| 21 | 44,863 | 3352 |

Even though the laser frequency ranges from 43,971cm⁻¹ to 44,863cm⁻¹, for a difference of 892cm⁻¹, the spectral shift remains relatively constant and shows no pronounced trend, proving that the observed peak is a Raman peak, not a fluorescence peak.

## Claims

1. A system for examining tissue or material comprising:
a fibre-optic catheter probe having a plurality of optical fibres (420) to provide a 2-D output insertable into a body lumen or tissue, the fibre-optic probe having a distal end (428) to be placed in proximity to said tissue or material;
a laser radiation source (492) coupled to a proximal end (448) of the fibre-optic probe to illuminate the tissue or material with a selected wavelength of light; and characterised by:
a spectrometer system (465) including an optional system between the proximal end (448) of the fibre optic probe and a charge coupled device (470);
said charge coupled device (470) having a 2-D array of detector elements that is optically coupled to the proximal and (448) of the fibre-optic probe by the optical system to image the 2-D Raman scattered radiation returning from the tissue or material in response to illumination from the laser radiation source (492); and
a controller connected to the charge coupled device (470) and a computer (480) for recording spectral data from the charge coupled device (470) and for forming a displayable image of the illuminated tissue.

2. The system of claim 1 wherein the light source (492) comprises an argon laser.

3. The system of either one of claims 1 and 2 wherein the fibre-optic probe comprises a laser catheter adapted for insertion into a vascular lumen.

4. The system of any preceding claim wherein the computer (480) arranged to analyze the detected radiation.

5. The system of claim 1 wherein the computer (480) compares the recorded spectra to stored spectra.

6. The system of claim 1 wherein said spectrometer system comprises a grating to direct light returning from the tissue or material and exiting the proximal end (448) of the probe onto the charge coupled device (470).

7. The system of claim 1 wherein said spectrometer system comprises a scanning interferometer (552b,545,546).

8. The system of claim 1 further comprising an optical filter.

9. The system of claim 1 further comprising a second detector optically coupled to the proximal end (448) of the probe.

10. The system of claim 1 wherein the charge coupled device (470) has at least as many pixels as there are optical fibres (420) in the probe.

11. The system of claim 1 in which the selected wavelength of light can be changed to a second wavelength thereby testing that the measured spectrum is a Raman signal.

12. A system as claimed in any preceding claim wherein the optical fibres (420) are arranged within the probe in a plurality of several separated columns such that light exiting from one column does not overlap that from any other column at the array of detector elements after dispersal by the spectrometer system.

13. A method of optically measuring vascular tissue or material by means of a laser fibre-optic catheter probe having a plurality of optical fibres (420) to provide a 2-D output such that a proximal end (448) of the probe can be coupled to a laser radiation source, the probe being positioned relative to vascular tissue or material such that a distal end (428) of the probe is in proximity with the vascular tissue or material to be measured; the method comprising:
coupling a laser radiation source (492) to the probe to irradiate the tissue or material at one or more selected wavelengths, the irradiation resulting in an emission of Raman scattered light from said tissue or material such that the emitted light is transmitted through the fibre-optic probe to the proximal end (448); and characterised by:
spectrally analysing the Raman scattered light from the vascular tissue or material; and
detecting the spectrally analyzed Raman scattered light returning from the tissue or material with a charge coupled device detector (470) to image the 2-D output from the probe and recording spectral data from the charge coupled device (470).

14. The method of claim 13 further comprising illuminating the tissue or material with ultraviolet light.

15. The method of claim 13 or 14 further comprising measuring Raman scattering from an hydroxyl group or a nitrogen-hydrogen bond within the tissue or material being measured.

16. The method of claim 15 further comprising changing the selected wavelength of light to a second wavelength thereby testing that the measured spectrum is a Raman signal.

## Patentansprüche

1. System zum Untersuchen von Gewebe oder Material, welches umfaßt:
eine faseroptische Kathetersonde mit einer vielzahl von Lichtleitfasern (420) zur Lieferung einer 2-D-Ausgabe, die in ein Körperlumen oder -gewebe einführbar ist, wobei die faseroptische Sonde ein fernes Ende (428) aufweist, das in der Nähe des Gewebes oder Materials angeordnet werden soll;
eine Laserstrahlungsquelle (492), die an ein nächstgelegenes Ende (448) der faseroptischen Sonde gekoppelt ist, um das Gewebe oder Material mit einer ausgewählten Lichtwellenlänge zu beleuchten; und gekennzeichnet durch:
ein Spektrometersystem (465), das zwischen dem nächstgelegenen Ende (448) der faseroptischen Sonde und einem ladungsgekoppelten Element (470) ein optisches System beinhaltet;
wobei das ladungsgekoppelte Element (470) eine 2-D-Anordnung von Detektorelementen aufweist, die durch das optische System an das nächstgelegene Ende (448) der faseroptischen Sonde optisch gekoppelt ist, um die vom Gewebe oder Material in Antwort auf die Beleuchtung von der Laserstrahlungsquelle (492) zurückkehrende, Raman-gestreute 2-D-Strahlung abzubilden; und
eine mit dem ladungsgekoppelten Element (470) verbundene Steuerung und einen Rechner (480) zum Aufzeichnen der Spektraldaten vom ladungsgekoppelten Element (470) und zum Bilden eines darstellbaren Bildes des beleuchteten Gewebes.

2. System nach Anspruch 1, bei dem die Lichtquelle (492) einen Argonlaser umfaßt.

3. System nach einem der Ansprüche 1 und 2, bei dem die faseroptische Sonde einen Laserkatheter umfaßt, der zum Einführen in ein Gefäßlumen ausgelegt ist.

4. System nach einem der vorhergehenden Ansprüche, bei dem der Rechner (480) so eingerichtet ist, daß er die erfaßte Strahlung analysiert.

5. System nach Anspruch 1, bei dem der Rechner (480) die aufgezeichneten Spektren mit gespeicherten Spektren vergleicht.

6. System nach Anspruch 1, bei dem das Spektrometersystem ein Gitter umfaßt, um vom Gewebe oder Material zurückkehrendes und aus dem nächstgelegenen Ende (448) der Sonde austretendes Licht auf das ladungsgekoppelte Element (470) zu richten.

7. System nach Anspruch 1, bei dem das Spektrometersystem ein Abtastinterferometer (552b, 545, 546) umfaßt.

8. System nach Anspruch 1, das weiterhin ein optisches Filter umfaßt.

9. System nach Anspruch 1, das weiterhin einen an das nächstgelegene Ende (448) der Sonde optisch gekoppelten, zweiten Detektor umfaßt.

10. System nach Anspruch 1, bei dem das ladungsgekoppelte Element (470) wenigstens so viele Pixel aufweist, wie es Lichtleitfasern (420) in der Sonde gibt.

11. System nach Anspruch 1, bei dem die ausgewählte Lichtwellenlänge zu einer zweiten Wellenlänge geändert werden kann, wodurch überprüft wird, daß das gemessene Spektrum ein Raman-Signal ist.

12. System nach einem der vorhergehenden Ansprüche, bei dem die Lichtleitfasern (420) innerhalb der Sonde in einer Vielzahl von verschiedenen getrennten Spalten angeordnet sind, so daß aus einer Spalte austretendes Licht das aus irgendeiner anderen Spalte bei der Anordnung von Detektorelementen nach der Streuung durch das Spektrometersystem nicht überlappt.

13. Verfahren zum optischen Messen von Gefäß-Gewebe oder Material mittels einer faseroptischen Laserkathetersonde mit einer Vielzahl von Lichtleitfasern (420) zur Lieferung einer 2-D-Ausgabe, so daß ein nächstgelegenes Ende (448) der Sonde an eine Laserstrahlungsquelle gekoppelt werden kann, wobei die Sonde relativ zum Gefäßgewebe oder Material so angeordnet wird, daß sich ein fernes Ende (428) der Sonde in der Nähe des zu messenden Gefäßgewebes oder Materials befindet; wobei das Verfahren umfaßt:
Koppeln der Laserstrahlungsquelle (492) an die Sonde, um das Gewebe oder Material bei einer oder mehreren ausgewählten Wellenlängen zu bestrahlen, wobei die Bestrahlung zu einer Aussendung von Raman-gestreutem Licht vom Gewebe oder Material führt, so daß das ausgesandte Licht über die faseroptische Sonde zum nächstgelegenen Ende (448) übertragen wird; und gekennzeichnet durch:
spektrales Analysieren des Raman-gestreuten Lichts vom Gefäßgewebe oder Material; und
Erfassen des vom Gewebe oder Material zurückkehrenden, spektral analysierten, Raman-gestreuten Lichts mit einem ladungsgekoppelten Element-Detektor (470), um die 2-D-Ausgabe aus der Sonde abzubilden und Spektraldaten vom ladungsgekoppelten Element (470) aufzuzeichnen.

14. Verfahren nach Anspruch 13, das weiterhin das Beleuchten des Gewebes oder Materials mit ultraviolettem Licht umfaßt.

15. Verfahren nach Anspruch 13 oder 14, das weiterhin das Messen der Raman-Streuung von einer Hydroxylgruppe oder einer Stickstoff-Wasserstoff-Bindung im gemessenen Gewebe oder Material umfaßt.

16. Verfahren nach Anspruch 15, das weiterhin das Ändern der ausgewählten Lichtwellenläge zu einer zweiten Wellenlänge umfaßt, wodurch überprüft wird, daß das gemessene Spektrum ein Raman-Signal ist.

## Revendications

1. Système pour examiner un tissu ou un matériau comprenant :
une sonde de cathéter à fibre optique comportant une pluralité de fibres optiques (420) pour obtenir une sortie en 2D, qui peut être insérée dans un orifice d'un corps ou un tissu, la sonde à fibre optique ayant une extrémité distale (428) destinée à être placée à proximité dudit tissu ou matériau ;
une source (492) de rayonnement laser couplée à une extrémité proximale (448) de la sonde à fibre optique pour éclairer le tissu ou matériau avec une longueur d'onde sélectionnée de lumière ; et caractérisé par :
un système (465) à spectromètre comportant un système optique entre l'extrémité proximale (448) de la sonde à fibre optique et un dispositif (470) à couplage de charge ;
ledit dispositif (470) à couplage de charge comportant un réseau en 2D d'éléments détecteurs, qui est couplé optiquement à l'extrémité proximale (448) de la sonde à fibre optique par le système optique pour visualiser le rayonnement dispersé de Raman en 2D renvoyé par le tissu ou matériau en réponse à un éclairage en provenance de la source (492) de rayonnement laser ; et
un dispositif de commande connecté au dispositif (470) à couplage de charge et un ordinateur (480) pour enregistrer des données spectrales en provenance du dispositif (470) à couplage de charge et pour former une image affichageable du tissu éclairé.

2. Système selon la revendication 1, dans lequel la source (492) de lumière comprend un laser à l'argon.

3. Système selon l'une ou l'autre des revendications 1 et 2, dans lequel la sonde à fibre optique comprend un cathéter à laser apte à une insertion dans un orifice vasculaire.

4. Système selon l'une quelconque des revendications précédentes, dans lequel l'ordinateur (480) est agencé pour analyser le rayonnement détecté.

5. Système selon la revendication 1, dans lequel l'ordinateur (480) compare les spectres enregistrés à des spectres stockés.

6. Système selon la revendication 1, dans lequel ledit système à spectromètre comprend un réseau pour diriger la lumière renvoyée par le tissu ou matériau et sortant de l'extrémité proximale (448) de la sonde sur le dispositif (470) à couplage de charge.

7. Système selon la revendication 1, dans lequel ledit système à spectromètre comprend un interféromètre à balayage (552b, 545, 546).

8. Système selon la revendication 1, comprenant en outre un filtre optique.

9. Système selon la revendication 1, comprenant en outre un deuxième détecteur couplé optiquement à l'extrémité proximale (448) de la sonde.

10. Système selon la revendication 1, dans lequel le dispositif (470) à couplage de charge comporte au moins autant de pixels qu'il y a de fibres optiques (420) dans la sonde.

11. Système selon la revendication 1, dans lequel la longueur d'onde sélectionnée de lumière peut être modifiée pour une deuxième longueur d'onde afin de tester que le spectre mesuré est un signal de Raman.

12. Système selon l'une quelconque des revendications précédentes, dans lequel les fibres optiques (420) sont agencées dans la sonde en une pluralité de plusieurs colonnes séparées de telle sorte que la lumière sortant d'une colonne ne chevauche pas celle d'une autre colonne quelconque au niveau du réseau d'éléments détecteurs après dispersion par le système à spectromètre.

13. Procédé de mesure optique d'un tissu vasculaire ou d'un matériau au moyen d'une sonde à cathéter à fibre optique à laser comportant une pluralité de fibres optiques (420) pour obtenir une sortie en 2D, de telle sorte qu'une extrémité proximale (448) de la sonde peut être couplée à une source de rayonnement laser, la sonde étant positionnée par rapport à un tissu vasculaire ou un matériau de telle sorte qu'une extrémité distale (428) de la sonde est à proximité du tissu vasculaire ou du matériau à mesurer ; le procédé comprenant :
un couplage d'une source (492) de rayonnement laser à la sonde pour irradier le tissu ou matériau à une ou plusieurs longueurs d'ondes sélectionnées, l'irradiation conduisant à une émission de lumière dispersée de Raman en provenance du tissu ou du matériau, de telle sorte que la lumière émise est transmise par l'intermédiaire de la sonde à fibre optique vers l'extrémité proximale (448) ; et caractérisé par :
une analyse spectrale de la lumière dispersée de Raman en provenance du tissu vasculaire ou matériau ; et
une détection de la lumière dispersée de Raman analysée spectralement, renvoyée par le tissu ou matériau, avec un détecteur (470) à couplage de charge pour visualier la sortie en 2D de la sonde et un enregistrement des données spectrales en provenance du dispositif (470) à couplage de charge.

14. Procédé selon la revendication 13, comprenant en outre l'éclairage du tissu ou matériau avec de la lumière ultraviolette.

15. Procédé selon la revendication 13 ou 14, comprenant en outre une mesure d'une dispersion de Raman à partir d'un groupe hydroxyle ou une liaison azote-hydrogène dans le tissu ou matériau mesurée.

16. Procédé selon la revendication 15, comprenant en outre une modification de la longueur d'onde sélectionnée de lumière pour une deuxième longueur d'onde afin de tester que le spectre mesuré est un signal de Raman.
